# EUROPÄISCHE PATENTSCHRIFT

(11) **EP 1 960 025 B1**
(45) Veröffentlichungstag und Bekanntmachung des Hinweises auf die Patenterteilung: **19.12.2018**
(21) Anmeldenummer: 06817710.4
(22) Anmeldetag: 09.11.2006
(51) Int. Cl.: A61M 16/08, A61M 16/00, A61M 16/20

(54) **SCHLAUCHSYSTEM FÜR BEATMUNGSGERÄTE**
FLEXIBLE CONDUIT SYSTEM FOR RESPIRATORY DEVICES
SYSTEME DE CONDUITS FLEXIBLES POUR APPAREILS RESPIRATOIRES

(30) Priorität: 16.12.2005 CH 19932005
(43) Veröffentlichungstag der Anmeldung: 27.08.2008
(73) Patentinhaber: Hamilton Medical AG, 7402 Bonaduz (CH)
(72) Erfinder: BRUNNER, Josef, CH-7000 Chur (CH)
(74) Vertreter: Ruttensperger Lachnit Trossin Gomoll
(86) Internationale Anmeldenummer: PCT/CH2006/000631
(87) Internationale Veröffentlichungsnummer: WO 2007/068132

(56) Entgegenhaltungen:
- WO-A-98/06449
- WO-A-03/055552
- WO-A-2004/084980
- WO-A2-03/033175
- FR-A1- 2 249 650
- US-A- 4 083 245
- US-A1- 2005 087 190

## Beschreibung

### Gebiet der Erfindung

Die Erfindung betrifft ein Schlauchsystem für Beatmungsgeräte, sowie ein Beatmungsgerät mit einem solchen Schlauchsystem.

### Stand der Technik

Für invasive mechanische Beatmung werden Zweischlauchsysteme verwendet. Zweischlauchsysteme besitzen einen Inspirationsschlauch und einen Exspirationsschlauch, die über ein Y-Stück an die Patientenmaske angeschlossen sind. Der Inspirationsschlauch ist anschliessend an einen das Beatmungsgerät vor Kontamination schützenden Inspirationsfilter angeschlossen. Zwischen einem geräteseitigen und einem patientenseitigen Inspirationsschlauchstück ist gegebenenfalls ein Befeuchtungsgerät angeordnet. Patientenseitig des Y-Stücks ist gegebenenfalls ein Flusssensor und über einen Zwischenschlauch der Tubus angeordnet.

Der an das Y-Stück anschliessende Exspirationsschlauch führt die Atemluft zurück zum Beatmungsgerät, und ist gegebenenfalls mit einer Wasserfalle ausgerüstet. Das Beatmungsgerät besitzt ein Inspirationsventil und ein Exspirationsventil, welche beide aktiv angesteuert werden. Zum Einatmen wird das Exspirationsventil geschlossen und das Inspirationsventil geöffnet, um einen Überdruck in der Lunge und den Schläuchen zu erhalten. Beim Ausatmen wird das Exspirationsventil geöffnet und das Inspirationsventil geschlossen, um ein möglichst widerstandsarmes Ausatmen sicherzustellen. Dank der aktiven Steuerung von Inspirationsventil und Exspirationsventil kann auch ein endexspiratorischer Druck dosiert werden.

Um bei Zweischlauchsystemen das tatsächliche Beatmungsvolumen feststellen zu können und die Eigenaktivität des Patienten feststellen zu können schaltet die Anmelderin zwischen das Y-Stück und die Maske einen Flusssensor gemäss US-A-4,083,245.

Für nicht-invasive mechanische Beatmung werden Einschlauch-Systeme (single limb breathing systems) verwendet. Einschlauch-Systeme zeichnen sich aus durch einen einzigen Beatmungsschlauch, der an einen das Beatmungsgerät vor Kontamination schützenden Inspirationsfilter anschliesst, und der die Distanz zwischen Beatmungsgerät und Patient überbrückt, sowie eine Maske. Es gibt geschlossene und offene Einschlauchsysteme. Geschlossene Einschlauchsysteme besitzen ein aktives Exspirationsventil, das meist über einen Luftdruckschlauch durch das Beatmungsgerät gesteuert ist. Es sitzt patientenseitig im Beatmungsschlauch. Das System ist oft mit einem Druclc-Messschlauch ausgerüstet, über den ein Druck der Beatmungsluft oder Ausatemluft beim Exspirationsventil gemessen werden kann, um die effektive Ventilation des Patienten berechnen zu können. Zwischen Exspirationsventil und Maske kann ein Zwischenschlauch vorhanden sein, damit das Exspirationsventil nicht direkt vor dem Gesicht des Patienten angeordnet ist. Der Patient wird vom Beatmungsgerät durch den Beatmungsschlauch hindurch beatmet. Beim Einatmen des Patienten ist das Exspirationsventil in einer Inspirationsstellung, in der ein im Schlauch vorhandener Überdruck in die Maske und die Patientenlunge gelangt. Eine Exspirationsöffnung ist geschlossen, damit in der Maske sich der gewünschte Überdruck der Beatmungsluft aufbauen kann. Beim Ausatmen ist das Exspirationsventil in einer Exspirationsstellung, in der eine Verbindung zwischen Schlauch und Maske verschlossen ist, aber die Ausatemluft mit möglichst geringem Widerstand durch die Exspirationsöffnung entweichen kann. Solche Einschlauchsysteme sind praktisch gleichwertig mit Zweischlauchsystemen. Sie unterscheiden sich von Zweischlauchsystemen praktisch lediglich darin, dass das Exspirationsventil patientennah angeordnet ist und daher aus dem Beatmungsgerät ausgelagert ist.

Solche Einschlauchsysteme haben den Nachteil, dass das an der Maske hängende Gewicht und die Gegenwart des patientenseitigen Exspirationsventils für den Patienten störend sind.

Um diesen Nachteil zu überwinden gibt es auch offene Einschlauchsysteme, die auf ein aktives Exspirationsventil verzichten. Der Beatmungsschlauch eines solchen offenen Systems hat eine vorgegebene Leckage. Diese Leckage ist durch ein Loch im Beatmungsschlauch definiert, welches Loch einen gewählten Querschnitt aufweist. Diese Leckage ist beim patientenseitigen Ende des Schlauches ausgebildet. Durch diese Öffnung entweicht die Ausatemluft. Die frische Beatmungsluft entweicht teilweise auch durch diese Öffnung. Daher muss bei solchen Einschlauchsystemen mehr Beatmungsluft aufbereitet und durch den Schlauch gefördert werden, als der Patient benötigt. Um die notwendige Menge an Beatmungsluft berechnen zu können, wird bei dieser Leckage der Druck im Schlauch gemessen, oder es wird eine rechnerische Annäherung aufgrund von vom Hersteller ermittelten, das Schlauchsystem charakterisierenden Kenndaten gemacht. Diese offenen Einschlauchsysteme sind angenehm für den Patienten, denn der Patient hat lediglich einen einzigen Schlauch und die Maske auf sich. Er kann jederzeit ausatmen, selbst gegen den Druck des Beatmungsgeräts, und er kann jederzeit Einatmen, selbst wenn das Beatmungsgerät keine Beatmungsluft liefert.

Bei offenen Einschlauchsystemen wird beim Ausatmen der Druck im Beatmungsschlauch durch das Beatmungsgerät kurzfristig abgelassen. Damit wird ein widerstandsarmes Ausatmen des Patienten ermöglicht. Dabei strömt verbrauchte Ausatemluft in den Beatmungsschlauch. Kurz vor Abschluss der Exspirationszeit wird der Beatmungsdruck im Beatmungsschlauch wieder aufgebaut. Während der Atempause bis zur nächsten Inspiration wird dadurch die im Schlauch vorhandene Ausatemluft durch die Leckage hinaus gepresst und so der Schlauch gespült.

Bei einem Wechsel von invasiver zu nicht-invasiver Beatmung wird in der Regel daher von einem Zweischlauchsystem auf ein offenes oder geschlossenes Einschlauchsystem umgestellt werden, um den Patienten vom schweren Zweischlauchsystem zu entlasten. Da Beatmungsgeräte für invasive Beatmung aber darauf angewiesen sind, dass der Exspirationsschlauch am Exspirationsventil angeschlossen ist, um den Beatmungsdruck im Schlauch und der Lunge kontrollieren zu können, ist es bisher erforderlich, mit dem Schlauchsystem auch das Beatmungsgerät zu wechseln.

Aus den Dokumenten FR 2249650 A1, WO 98/06449 A1, WO 03/055552 A1, WO2004/084980 A1, US 2005/087190 A1 und WO 03/033175 A2 sind verschiedene Schlauchsysteme für Beatmungsgeräte bekannt.

### Aufgabe der Erfindung

Es ist daher die Aufgabe der vorliegenden Erfindung, zu ermöglichen, ein Beatmungsgerät, das für die invasive Beatmung mit einem aktiv gesteuerten Inspirationsventil und einem aktiv gesteuerten Exspirationsventil ausgerüstet ist, zur nicht-invasiven Beatmung mit einem für den Patienten angenehm leichten Schlauchsystem, insbesondere einem handelsüblichen Schlauch eines Einschlauchsystems zu verwenden.

### Lösung der Aufgabe

Diese Aufgabe wird erfindungsgemäss gelöst durch die Merkmale des Anspruchs 1.

Demgemäss ist ein dreiarmiges, d.h. Y-förmig ausgebildetes Schlauchsystem für ein für invasive Beatmung geeignetes Beatmungsgerät, das ein aktiv gesteuertes Inspirationsventil und ein aktiv gesteuertes Exspirationsventil aufweist, mit einem Inspirationsschlaucharm zum Anschliessen an das Inspirationsventil des Beatmungsgeräts, einem Exspirationsschlaucharm zum Anschliessen an das Exspirationsventil des Beatmungsgeräts, und einem Beatmungsschlaucharm zum Anschluss an eine Maske oder einen Tubus ausgebildet. Es weist zudem einen Flusssensor auf, der zum Messen des Inspirationsvolumens und des Exspirationsvolumens am Beätmungsschlaucharm angeordnet oder anzuordnen ist. Erfindungsgemäss ist nun bei diesem Schlauchsystem im Beatmungsschlaucharm eine definierte Leckage ausgebildet und der Flusssensor zwischen dieser Leckage und der Maske oder dem Tubus angeordnet oder anzuordnen.

Die Leckage ermöglicht den Beatmungsschlaucharm mit einem grossen Volumen, d.h. mit einer grossen Länge auszubilden. Daher kann ein einziger Schlauch an die Maske oder den Tubus angeschlossen werden, so dass sich der Patient fühlt, als wäre er an einem offenen Einschlauchsystem angeschlossen. Der Patient muss kein störendes patientennahes Exspirationsventil und keine zwei Schläuche in Kauf nehmen. Lediglich gerätenahe ist das Schlauchsystem in den Inspirationsschlaucharm und den Exspirationsschlaucharm aufgeteilt.

Die Leckage hat den Vorteil, dass der Patient auch atmen kann, falls das Gerät aus irgendeinem Grund keine Beatmungsluft liefert. Der Patient kann auch jederzeit sich gegen die mechanische Beatmung wehren. Die Beatmungsluft strömt dann einfach durch die Leckage. Der Patient kann auch jederzeit ausatmen, also auch bei geschlossenem Exspirationsventil. Diese Freiheiten des Patienten reduzieren das beengende Gefühl einer mechanischen Beatmung. Dank der Leckage wird der Beatmungsschlauch vor jeder Inspirationsphase gespült. Die im Beatmungsschlauch vorhandene Ausatemluft wird dabei durch die Leckage ausgepresst. Bis der Beatmungsdruck erreicht wird, mit welchem dem Patienten die Lunge gefüllt wird, ist praktisch der gesamte Inhalt des Beatmungsschlauchs aus diesem hinaus geströmt. Die Lunge wird daher mit frischer Beatmungsluft gefüllt.

Moderne Beatmungsgeräte besitzen Algorithmen, dank denen die zu liefernde Luftmenge, der zu liefernde Druck und die Atemfrequenz aufgrund der mit dem Flusssensor gemessenen Parameter berechnet werden kann. Verluste durch eine unbestimmte und/oder die definierte Leckage werden durch das Beatmungsgerät kompensiert.

Der Anschluss des offenen Beatmungsschlauchs über einen Exspirationsschlaucharm an ein Exspirationsventil ermöglicht ein rasches, widerstandsarmes Ausatmen.

Zweckmässigerweise ist das Schlauchsystem zusammengesetzt. Die Bestandteile sind dann ein Inspirationsschlauch oder Inspirationsschlauchset, ein Exspirationsschlauch oder Exspirationsschlauchset, ein Y-Stück, ein Beatmungsschlauch oder Beatmungsschlauchset eines offenen Einschlauchsystems und der Flusssensor. Gegebenenfalls gehört ein Anschlussschlauch zwischen Flusssensor und Maske oder Tubus zum Schlauchsystem. Unter Umständen gehört auch die Maske oder der Tubus zum Schlauchsystem.

Der Flusssensor besteht vorteilhaft aus einem Gehäuse, das einen Gaseinlassstutzen und einen Gasauslassstutzen aufweist, bei dem der Innenraum des Gehäuses zwischen dem Einlassstutzen und dem Auslassstutzen durch eine Blendenmembran in zwei Zonen geteilt ist. In jeder dieser Zonen ist eine Druckmessvorrichtung oder ein Anschluss an eine Druckmessvorrichtung vorhanden. Die Blendenmembran besteht vorteilhaft aus einem elastischen Werkstoff. In ihr ist eine Öffnung und eine mit der Blendenmembran einstückig ausgebildete Klappe vorhanden, die in Form und Grösse der Öffnung entspricht und scharnierartig mit der Blendenmembrane verbunden ist. Der Rand der Öffnung verläuft vorteilhaft divergierend von der scharnierartigen Verbindung weg, weist in Abstand zur scharnierartigen Verbindung einen Richtungswechsel auf, um gegenüber der scharnierartigen Verbindung eine Stelle geringster Breite der Öffnung zu bilden. Die Klappe ist zweckmässigerweise um die scharnierartige Verbindung verschwenkbar ausgebildet und ist bei hohem Gasdurchsatz vor eine in die ausströmungsseitige Zone mündende Druckmessöffnung verschwenkbar.

Die einzige Klappe besitzt eine Elastizität und die einzige Öffnung besitzt eine Form, welche gewährleisten, dass der Widerstand der Öffnung über einen grossen Durchsatzbereich konstant ist.

Derartige Flusssensoren sind bekannt und werden durch die Anmelderin bei herkömmlichen Zweischlauchsystemen mit Erfolg eingesetzt.

Ein zur invasiven Beatmung geeignetes Beatmungsgerät besitzt ein aktiv gesteuertes Inspirationsventil und üblicherweise ein aktiv gesteuertes Exspirationsventil, und benötigt ein dreiarmiges Schlauchsystem zum Y-formigen Verbinden von Inspirationsventil, Patient und Exspirationsventil. Es ist bekannt, solche Schlauchsysteme mit einem patientennah anzuordnenden Flusssensor zu bestücken. Ein solches Beatmungsgerät mit Schlauchsystem zeichnet sich nun aber neu dadurch aus, dass in einem zum Patienten hin gerichteten Beatmungsschlaucharm eine definierte Leckage vorhanden ist, und der Flusssensor zwischen der Leckage und einer Maske, oder einem Tubus, angeordnet ist. Die Vorteile dieser Leckage sind oben beschrieben.

Das Beatmungsgerät wird entsprechend neuartig betrieben. Die Beatmungsluft wird in bekannte Weise über ein aktiv gesteuertes Inspirationsventil und einen Inspirationsschlauch einem Beatmungsschlauch und einer patientenseitigen Beatmungsmaske oder einem Tubus zugespiesen. Die Ausatemluft wird wenigstens teilweise in ebenfalls bekannter Weise über den Beatmungsschlauch, einen Exspirationsschlauch und ein aktiv gesteuertes Exspirationsventil abgelassen. Die Atemfrequenz und das Atemvolumen werden mittels eines patientenseitigen Flusssensors überwacht. Es werden Beatmungsdruck, endexspiratorischer Druck, Beatmungsfrequenz und Volumen der Beatmungsluft unter anderem aufgrund der durch den Flusssensor gelieferten Parameter geregelt. Bei dieser Regelung werden im Schlauchsystem gegebenenfalls vorhandene Leckagen mit Hilfe einer Flussmessung im Beatmungsgerät und der Flussmessung mittels des patientenseitigen Flusssensors quantitativ bestimmt und durch das Beatmungsgerät kompensiert. Es ist indes neu, dass im Beatmungsschlauch eine definierte Leckage auf der vom Patienten abgewandten Seite des Flusssensors vorgesehen ist. Das mit einem Exspirationsventil ausgerüstete Beatmungsgerät presst bei dem hier beschriebenen Verfahren in der Atempause zwischen dem Ende der Ausatemphase und dem Beginn der Einatemphase im Beatmungsschlauch vorhandenes Gas durch die Leckage aus dem Schlauchsystem hinaus.

Zum mechanischen Beatmen eines Patienten mit einem für invasive Beatmung geeigneten Beatmungsgerät wird in bekannter Weise mit Hilfe eines patientenseitigen Flusssensors die Atemfrequenz, der Beatmungsdruck, der endexspiratorische Druck und das Beatmungsvolumen überwacht. Aufgrund der vom Flusssensor ermittelten Parameter und mit Hilfe des Gasmischers, des Inspirationsventils und des Exspirationsventils werden der Beatmungsdruck, das Beatmungsvolumen und der endexspiratorischen Druck patientengerecht geregelt. Gegebenenfalls vorhandene Leckagen werden kompensiert, indem der Gasmischer, das Inspirationsventil und das Exspirationsventil aktiv gesteuert werden. Ist jedoch erfindungsgemäss im Beatmungsschlaucharm auf der patientenabgewandten Seite des patientennahen Flusssensors eine definierte Leckage vorgesehen, wird die Ausatemluft teilweise durch diese Leckage ausgeblasen, bevor die Patientenlunge mit frischer Beatmungsluft gefüllt wird. Dies hat den Vorteil, dass ein bekanntes Einschlauchsystem mit einem für Zweischlauchsysteme konzipierten Beatmungsgerät verwendet werden kann, und der Patient daher ohne Gerätewechsel zwischen einer invasiven und einer nicht-invasiven Beatmung gewechselt werden kann.

### Kurzbeschreibung der Figuren

- Fig. 1: zeigt schematisch den bekannten Stand der Technik bezüglich Zweischlauchsystemen für invasive Beatmungsgeräte.
- Fig. 2: zeigt schematisch den bekannten Stand der Technik bezüglich offenen Einschlauchsystemen.
- Fig. 3: zeigt schematisch den Stand der Technik bezüglich Flusssensor.
- Fig. 4: zeigt schematisch das erfindungsgemässe Schlauchsystem.

Detailliert Beschreibung der in den Figuren dargestellten Ausführungsbeispiele

### Stand der Technik

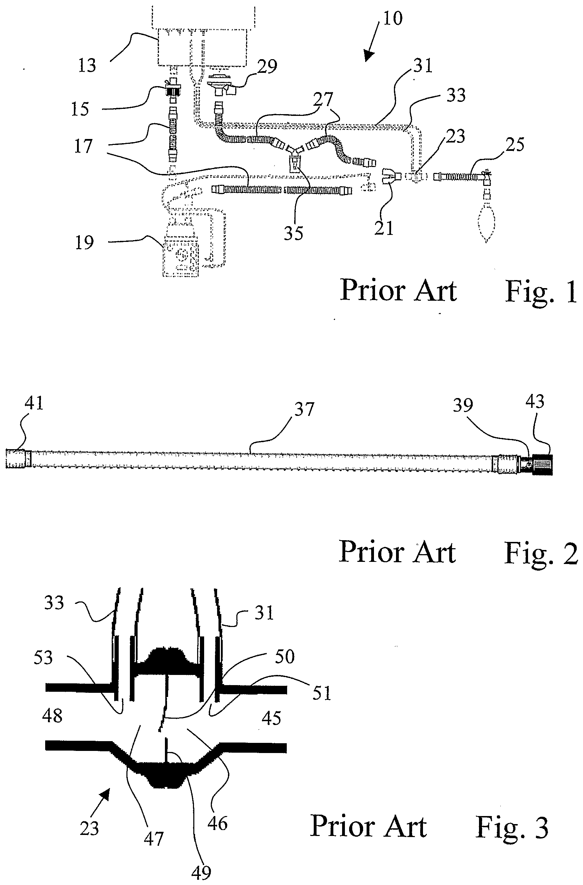

Wie eingangs beschrieben gibt es in Stand der Technik Einschlauchsysteme und Zweischlauchsysteme. Das in Figur 1 dargestellte bekannte Zweischlauchsystem ist für invasive Beatmung geeignet und konzipiert. Dieses Zweischlauchsystem ist daher an einem Beatmungsgerät 13 anzuschliessen, das für die invasive Beatmung geeignet ist. Ein solches invasives Beatmungsgerät 13 hat zwei aktiv gesteuerte Ventile, nämlich das Inspirationsventil (nicht dargestellt, da es im Innern des Geräts angeordnet ist) und das Exspirationsventil 29. Ein an einem Geräteausgang über einen Filter 15 angeschlossenes Inspirationsschlauch-Set 17 und ein am Exspirationsventil 29 angeschlossenes Exspirationsschlauch-Set 27 sind an ihren vom Beatmungsgerät 13 abgewandten Enden an einem Y-Stück 21 angeschlossen. Das Y-Stück 21 fasst die beiden Schläuche 17 und 27 zusammen und verbindet sie mit einem zum Patienten führenden Schlauch 25. Patientenseitig am Y-Stück ist ein Flusssensor 23 angeordnet. Der Flusssensor 23 ist über zwei Luftleitungen 31,33 mit dem Beatmungsgerät 13 verbunden. In den Schlauch-Sets 17 und 27 können Zubehörgeräte zwischengesteckt sein. Am Inspirationsschlauch 17 ist zweckmässigerweise ein Befeuchtungsgerät 19 angeschlossen. Im Exspirationsschlauch 27 ist zweckmässigerweise eine Wasserfalle 35 vorgesehen. Die Atemluft des Patienten strömt lediglich innerhalb des Verbindungsschlauchs 25 und des Flusssensors 23 in beide Richtungen. Der Inhalt des Verbindungsschlauchs 25 und des Flusssensors 23 müssen daher als serieller Totraum in eine Berechnung des notwendigen Atemvolumens einfliessen.

Für offene Einschlauchsysteme werden Beatmungsschläuche 37 verwendet. Ein solcher bekannter Beatmungsschlauch 37 (Fig. 2) wird über seine gesamte Länge in beide Richtungen von der Atemluft durchströmt. Damit dieser Beatmungsschlauch 37 nicht einen sehr grossen seriellen Totraum bildet, besitzt er eine Leckage 39. Durch die Leckage strömt beim Ausatmen und während der Atempause nach dem Ausatmen, nämlich bis der notwendige Beatmungsdruck wieder aufgebaut ist, praktisch die gesamte verbrauchte Atemluft aus. Aus der Leckage 39 strömt indes auch ein Teil der frischen Beatmungsluft aus. Es ist bei der Einrichtung des Beatmungsschlauchs 37 zwischen dem Patienten und dem Beatmungsgerät wichtig darauf zu achten, dass die Leckage patientennah ist um den seriellen Totraum möglichst klein zu halten. Das leckageferne Ende 41 des Beatmungsschlauchs 37 ist daher an das Beatmungsgerät anzuschliessen, das leckagenahe Ende 43 ist direkt oder über einen Verbindungsschlauch 25 an eine Maske anzuschliessen. Solche offenen Beatmungssysteme werden bisher lediglich für die nicht-invasive Maskenbeatmung eingesetzt. Es erschien bisher als unmöglich, einen Schlauch eines Einschlauchsystems an einem Gerät für ein Zweischlauchsystem anzuschliessen.

In Figur 3 ist ein schematischer Schnitt durch den bekannten Flusssensor 23 dargestellt. Der Flusssensor 23 besitzt einen Einlassstutzen 45, eine erste Kammer 46, eine zweite Kammer 47 und einen Auslassstutzen in einem Gehäuse. Die erste und die zweite Kammer 46,47 sind getrennt mittels einer Membrane 49. Diese Membrane 49 besitzt eine Öffnung und eine die Öffnung ausfüllende Klappe 50. Die Klappe 50 ist wie eine Pendeltüre verschwenkbar ausgebildet, so dass sie in die eine oder die andere Kammer hineinstehen kann, je nachdem, in welche Richtung der Atemluftfluss gerichtet ist. Der Flusssensor 23 ist symmetrisch ausgebildet, so dass er in beide Richtungen den Druckabfall messen kann, der in Folge des Durchströmens der Atemluft durch die Öffnung entsteht. Die Drücke in den Kammern werden über die Luftschläuche 31, 33 dem Beatmungsgerät zugeführt und im Beatmungsgerät gemessen. Aufgrund dieses Druckabfalls kann das Beatmungsgerät die Druckverhältnisse in der Atemluft und das Beatmungsvolumen berechnen. Diese Berechnungen sind praktisch unabhängig davon, wie gross eine Leckage zwischen dem Flusssensor und dem Beatmungsgerät 13 ist, sehr aussagekräftig.

### Erfindungsgemässes Ausführungsbeispiel

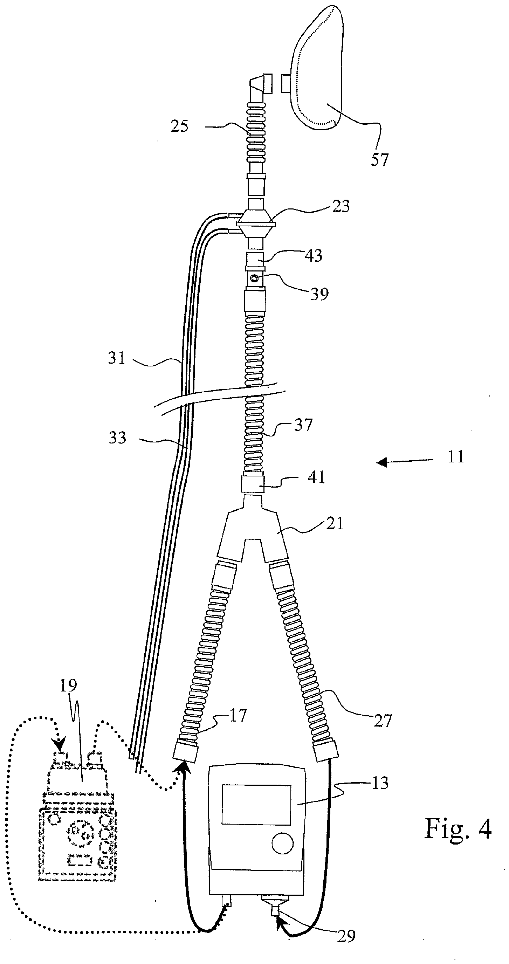

In Figur 4 ist nun das erfindungsgemässe Schlauchsystem 11 dargestellt. Die Bestandteile sind jeweils bereits bekannt. Die Zusammensetzung ist jedoch neu und weist entscheidende Vorteile auf. Der Inspirationsschlauch 17 und der Exspirationsschlauch 27 sind kurze Schlauchstücke, wie sie beispielsweise im Schlauchsystem 10 gemäss Figur 1 zwischen dem Filter 15 und dem Befeuchtungsgerät 19 verwendet sind. Ihre Länge muss lediglich erlauben, dass beide Schläuche mit den einen Enden an das Beatmungsgerät anschlossen werden können. In diesem Bereich zwischen Beatmungsgerät 13 und Y-Stück 21 kann auch ein Befeuchtungsgerät 19 vorgesehen sein. Das Y-Stück, das diese beiden geräteseitigen Schläuche 17, 27 zusammenführt und mit dem Beatmungsschlauch 37 verbindet, kann identisch mit dem bisher bekannten Y-Stück sein. Der daran anschliessende Beatmungsschlauch 37 ist ein Schlauch gemäss Figur 2 und ebenfalls bekannt. Ebenso bekannt sind der Flusssensor 23, der patientenseitig am Beatmungsschlauch 37 angeordnet ist. Die erfindungsgemässe Zusammenstellung bekannter Beatmungsschlauch-Komponenten zeichnet sich daher dadurch aus, dass ein Y-förmiges Schlauchsystem gebildet ist, das zwei vorzugsweise kurze Arme 17,27 aufweist, die an einem invasiven Beatmungsgerät 13 angeschlossen werden können. Der dritte Arm ist ein langer Beatmungsschlauch 37 mit einer patientenseitig vorgesehenen, definierten Leckage 39. Weiter ist ein Flusssensor 23 und zweckmässigerweise ein Verbindungsschlauch 25 an den Beatmungsschlauch angeschlossen. Am Verbindungsschlauch 25 kann eine Maske 57, in gewissen Fällen aber auch ein Tubus angeordnet sein.

Zum Schutz des Beatmungsgeräts kann ein Filter 15 vorgesehen sein. Dieser Filter kann zwischen dem Gerät 13 und dem Inspirationsschlauch 17 vorgesehen sein.

Dieses Schlauchsystem 11 hat gegenüber den bekannten Schlauchsystemen entscheidende Vorteile:
- Es kann ein für invasive Beatmung konzipiertes Gerät mit diesem erfindungsgemässen Schlauchsystem auch für nicht-invasive Beatmung verwendet werden.
- Beim Wechsel von invasiver zu nicht-invasiver Beatmung muss lediglich das Zweischlauchsystem mit dem Tubus gegen ein erfindungsgemässes Y-Schlauchsystem mit einer Maske ausgetauscht werden.
- Beim Wechsel von nicht-invasiver zu invasiver Beatmung kann rasch gehandelt werden, da das Beatmungsgerät bereits für eine invasive Beatmung geeignet ist.
- Es können Standard-Komponenten verwendet werden.
- Bei nicht-invasiver Beatmung ist das Schlauchsystem minimal bezüglich Gewicht und Behinderung des Patienten.
- Bei nicht-invasiver Beatmung erlaubt das Schlauchsystem maximale Bewegungsfreiheit, Atemfreiheit, Sicherheit bei Fehlern, minimaler Ausatemwiderstand, maximale Kontrolle durch patientennahe Überwachung.
- Das definierte Leck (39) muss nicht mit Hilfe eines Manövers gemessen werden, da der proximale Flusssensor (23) das tatsächlich abgegebene Beatmungsvolumen misst und die Leckage im Schlauchsystem automatisch kompensiert.

Eine Kalibrierung des Flusssensors kann in herkömmlicher Weise geschehen, wobei während dem Kalibrierungsmanöver die Leckage, z.B. mit einem auf die Öffnung gepressten Finger, verschlossen sein muss.

## Patentansprüche

1. Dreiarmiges Schlauchsystem (11) für ein für invasive Beatmung geeignetes Beatmungsgerät (13), welches Beatmungsgerät ein aktiv gesteuertes Inspirationsventil und ein aktiv gesteuertes Exspirationsventil (29) aufweist,
welches Schlauchsystem (11) mit
- einem Inspirationsschlaucharm (17), welcher zum Anschließen an das aktiv gesteuerte Inspirationsventil des Beatmungsgeräts (13) ausgebildet ist, und
- einem Exspirationsschlaucharm (27), welcher zum Anschließen an das aktiv gesteuerte Exspirationsventil (29) des Beatmungsgeräts (13) ausgebildet ist, und
- einem Beatmungsschlaucharm (37), welcher zum Anschluss an eine Maske (57) oder einen Tubus ausgebildet ist,
- sowie einen Flusssensor (23) aufweist, der zum Messen des Inspirationsvolumens und des Exspirationsvolumens am Beatmungsschlaucharm (37) angeordnet oder anzuordnen ist,
**dadurch gekennzeichnet,**
- **dass** im Beatmungsschlaucharm (37) eine definierte Leckage (39) ausgebildet ist und
- **dass** der Flusssensor (23) zwischen Leckage (39) und Maske (57) oder Tubus angeordnet oder anzuordnen ist.

2. Schlauchsystem nach Anspruch 1,
**dadurch gekennzeichnet, dass** das Schlauchsystem (11) einen Inspirationsschlauch oder ein Inspirationsschlauchset, einen Exspirationsschlauch oder ein Exspirationsschlauchset, ein Y-Stück (21), einen Beatmungsschlauch oder ein Beatmungsschlauchset eines offenen Einschlauchsystems, also einen Beatmungsschlauch oder ein Beatmungsschlauchset mit der definierten Leckage (39) und eine Maske (57) und/oder einen Tubus umfasst.

3. Schlauchsystem nach Anspruch 1 oder 2,
**dadurch gekennzeichnet, dass** der Flusssensor (23) aus einem Gehäuse besteht, das einen Gaseinlassstutzen (45) und einen Gasauslassstutzen (48) aufweist, der Innenraum des Gehäuses zwischen dem Einlassstutzen (45) und dem Auslassstutzen (48) durch eine Blendenmembran (49) in zwei Zonen (46, 47) geteilt ist, und in jeder dieser Zonen eine Druckmessvorrichtung oder ein Anschluss (51, 53) an eine Druckmessvorrichtung vorhanden ist.

4. Schlauchsystem nach Anspruch 3,
**dadurch gekennzeichnet, dass** die Blendenmembran (49) aus einem elastischen Werkstoff besteht, darin eine Öffnung ausgebildet ist und dass eine mit der Blendenmembran einstückig ausgebildete Klappe (50), die in Form und Größe der Öffnung entspricht und scharnierartig mit der Blendenmembrane (49) verbunden ist.

5. Schlauchsystem nach Anspruch 4,
**dadurch gekennzeichnet, dass** der Rand der Öffnung von der scharnierartigen Verbindung weg divergierend verläuft, in Abstand zur scharnierartigen Verbindung einen Richtungswechsel aufweist, um gegenüber der scharnierartigen Verbindung eine Stelle geringster Breite der Öffnung zu bilden.

6. Schlauchsystem nach einem der Ansprüche 4 bis 5,
**dadurch gekennzeichnet, dass** die Klappe (50) um die scharnierartige Verbindung verschwenkbar ausgebildet ist und bei hohem Gasdurchsatz vor eine in die ausströmungsseitige Zone (47) mündende Druckmessöffnung (53) verschwenkbar ist.

7. Schlauchsystem nach einem der Ansprüche 4 bis 6,
**dadurch gekennzeichnet, dass** in der Blendenmembran (49) eine einzige Klappe (50) und eine einzige Öffnung ausgebildet ist.

8. Schlauchsystem nach einem der Ansprüche 4 bis 5,
**dadurch gekennzeichnet, dass** die Formgebung und die Elastizität der Klappe (50) derart ausgelegt ist, dass der Widerstand der Öffnung über einen großen Durchsatzbereich konstant ist.

9. Zur invasiven Beatmung geeignetes Beatmungsgerät (13) mit einem aktiv gesteuerten Exspirationsventil (29) und einem aktiv gesteuerten Inspirationsventil, und einem dreiarmigen Schlauchsystem (11) nach einem der vorhergehenden Ansprüche zum Verbinden von Inspirationsventil und Exspirationsventil (29) mit einer Patientenmaske (57) oder einem Tubus, wobei der Flusssensor (23) patientennah zwischen der Leckage (39) und der Maske (57) oder dem Tubus angeordnet ist.

## Claims

1. A three-arm tube system (11) for a ventilation appliance (13) suitable for invasive ventilation, wherein the ventilation appliance comprises an actively controlled inhalation valve and an actively controlled exhalation valve (29), wherein the tube system (11) comprises:
- an inhalation tube arm (17) configured for connecting to the actively controlled inhalation valve of the ventilation appliance (13); and
- an exhalation tube arm (27) configured for connecting to the actively controlled exhalation valve (29) of the ventilation appliance (13), and
- a ventilation tube arm (37) configured for connecting to a mask (57) or a respiratory tube;
- as well as a flow sensor (23) configured to be arranged or arranged at the ventilation tube arm (37) for measuring the inhalation volume and the exhalation volume,
**characterized in that,**
- a defined leakage (39) is formed in the ventilation tube arm (37), and
- the flow sensor (23) is arranged or is to be arranged between the leakage (39) and the mask (57) or the respiratory tube.

2. The tube system according to claim 1,
**characterized in that** the tube system (11) comprises an inhalation tube or an inhalation tube set, an exhalation tube or an exhalation tube set, a Y piece (21), a ventilation tube or a ventilation tube set of an open one-tube system, i.e. a ventilation tube or a ventilation tube set with the defined leakage (39) and a mask (57) and/or a respiratory tube.

3. The tube system according to claim 1 or 2,
**characterized in that** the flow sensor (23) consists of a housing having a gas inlet nozzle (45) and a gas outlet nozzle (48), the interior of the housing being divided into two zones (46, 47) between the inlet nozzle (45) and the outlet nozzle (48) by a baffle membrane (49), and **in that** a pressure measuring device, or a connection (51, 53) to a pressure measuring device is provided at each of these zones.

4. The tube system according to claim 3,
**characterized in that** the baffle membrane (49) is comprised of an elastic material, that an opening is formed therein, and that a flap (50) formed as one piece with the baffle membrane, corresponding to the shape and size of the opening, is connected to the baffle membrane (49) in the manner of a hinge-type connection.

5. The tube system according to claim 4,
**characterized in that** the edge of the opening extends divergently away from the hinge-type connection and has a directional change at a distance to the hinge-type connection in order to form a point of the lowest width of the opening opposite to the hinge-type connection.

6. The tube system according to one of claims 4 to 5,
**characterized in that** the flap (50) is adapted to pivot about the hinge-type connection and can be pivoted to the front of the opening ending (53) leading into the zone on the outflow side (47) in case of a high gas throughput.

7. The tube system according to one of claims 4 to 6,
**characterized in that** a single flap (50) and a single opening are included in the baffle membrane (49).

8. The tube system according to one of claims 4 to 5,
**characterized in that** the shape and the elasticity of the flap (50) are configured such that the resistance of the opening is constant across a large throughput range.

9. A ventilation appliance (13) suitable for invasive ventilation with an actively controlled exhalation valve (29) and an actively controlled inhalation valve, and a three-arm tube system (11) according to one of the preceding claims for connecting the inhalation valve and the exhalation valve (29) to a patient's mask (57), or a respiratory tube, wherein the flow sensor (23) is arranged adjacent to a patient between the leakage (39) and the mask (57) or the respiratory tube.

## Revendications

1. Un système de tuyaux à trois branches (11) pour un appareil respiratoire (13) approprié pour la ventilation invasive, l'appareil respiratoire comprenant une vanne d'inspiration contrôlée de manière active et une vanne d'expiration contrôlée de manière active (29),
le système de tuyaux à trois branches (11) comprenant :
- une branche de tuyau d'inspiration (17) adaptée pour la connexion avec la vanne d'inspiration contrôlée de manière active de l'appareil respiratoire (13), et
- une branche de tuyau d'expiration (27) adaptée pour la connexion avec la vanne d'expiration (29) contrôlée de manière active de l'appareil respiratoire (13), et
- une branche de tuyau de ventilation (37) adaptée pour la connexion à un masque (57) ou un tube,
- ainsi qu'un capteur de flux (23) arrangé ou à arranger pour mesurer le volume d'inspiration ou le volume d'expiration a la branche de tuyau de ventilation (37),
**caractérisé en ce que,**
- une fuite définie (39) est formée dans la branche de tuyau de ventilation (37), et
- le capteur de flux (23) est arrangé ou à arranger entre la fuite (39) et le masque (57) ou le tube.

2. Le système de tuyaux selon la revendication 1,
**caractérisé en ce que** le système de tuyaux (11) comprend un tuyau d'inspiration ou un ensemble de tuyaux d'inspiration, un tuyau d'expiration ou un ensemble de tuyaux d'expiration, une pièce Y (21), un tuyau de ventilation ou un ensemble de tuyau de ventilation d'un système ouvert à tuyau simple, i.e. un tuyau de ventilation ou un ensemble de tuyau de ventilation avec la fuite définie (39) et un masque (57) et/ou un tube.

3. Le système de tuyaux selon les revendications 1 ou 2,
**caractérisé en ce que** le capteur de flux (23) consiste en un boîtier comprenant un raccord d'entrée de gaz (45) et un raccord de sortie de gaz (48), l'intérieur du boîtier étant entre le raccord d'entrée de gaz (45) et le raccord de sortie de gaz (48) divisé en deux zones (46, 47) par une membrane à orifice (49) et **en ce que** dans chacune desdites zones il y a un dispositif de mesure de la pression ou une connexion (51, 53) à un dispositif de mesure de la pression.

4. Le système de tuyaux selon la revendication 3,
**caractérisé en ce que** la membrane à orifice (49) est faite d'un matériau flexible, qu'un orifice est formé là-dedans et qu'un clapet (50) formé en une pièce avec membrane à orifice correspondant à l'orifice en ce qui concerne la forme et les dimensions est connecté à la membrane à orifice (49) à la manière d'une charnière.

5. Le système de tuyaux selon la revendication 4,
**caractérisé en ce que** le bord de l'orifice s'étend divergeant de la connexion à la manière d'une charnière et présente un changement de direction avec une distance à la connexion à la manière d'une charnière pour former un endroit avec la plus petite largeur de l'orifice opposé à la connexion à la manière d'une charnière.

6. Le système de tuyaux selon une des revendications 4 à 5,
**caractérisé en ce que** le clapet (50) est adapté de manière pivotante autour de la connexion à la manière d'une charnière et peut, lors d'un grand débit de gaz, être pivoté devant un orifice de mesure de pression (53) menant dans la zone (47) du côté d'écoulement de sortie.

7. Le système de tuyaux selon une des revendications 4 à 6,
**caractérisé en ce qu'**un seul clapet (50) et un seul orifice sont formés dans la membrane à orifice (49).

8. Le système de tuyaux selon une des revendications 4 à 5,
**caractérisé en ce que** la forme et la flexibilité du clapet (50) sont de sorte que la résistance de l'orifice est constante pour une grande gamme de débit.

9. Un appareil respiratoire (13) approprié pour la ventilation invasive avec une vanne d'expiration (29) contrôlée de manière active et une vanne d'inspiration (29) contrôlée de manière active et un système de tuyaux à trois branches (11) selon une des revendications précédentes pour connecter la vanne d'inspiration et la vanne d'expiration (29) à un masque de patient (57) ou un tube, le capteur de flux (23) étant arrangé proche du patient entre la fuite (39) et le masque (57) ou le tube.
